# EUROPEAN PATENT APPLICATION

(11) **EP 4 640 266 A1**
(43) Date of publication of application: **29.10.2025**
(21) Application number: 23907148.3
(22) Date of filing: 21.12.2023
(51) Int. Cl.: A61M 25/14, A61M 25/092

(54) **CATHETER**

(30) Priority: 21.12.2022 JP 2022204052
(71) Applicant: Nipro Corporation, Osaka 566-8510 (JP)
(72) Inventor: AKIHAMA, Hidenobu, Settsu-shi, Osaka 566-8510 (JP); WATANABE, Hiroki, Settsu-shi, Osaka 566-8510 (JP); OTSUKA, Chiaki, Settsu-shi, Osaka 566-8510 (JP); KANKE, Ryota, Settsu-shi, Osaka 566-8510 (JP)
(74) Representative: Slingsby Partners LLP
(86) International application number: PCT/JP2023/045998
(87) International publication number: WO 2024/135788

(57) **Abstract**

The present invention provides a catheter having a novel structure that solves problems such as local stiffness at a portion where an operation wire is connected. A catheter 10 includes a main lumen 26 and an operation lumen 28, and is operable as a result of an operation wire 48 inserted through the operation lumen 28 being fixed on a distal end side. A distal end tube 46 is fastened in a butted state to a periphery of a distal end opening of the main lumen 26. The operation wire 48 extending out of a distal end opening of the operation lumen 28 is adhered to an outer circumferential surface of the distal end tube 46.

## Description

### TECHNICAL FIELD

The present invention relates to a catheter and a catheter assembly adapted to be inserted into somatic lumens to perform various medical procedures.

### BACKGROUND ART

Conventionally, a catheter has been known to be inserted into somatic lumens such as blood vessels, an abdominal cavity, and the like to perform minimally invasive medical treatments on internal lesions. The catheter is, for example, tubular in shape including a lumen, and has been disclosed in PCT Japanese Translation Patent Publication No. JP-A-2017-536158 (Patent Document 1), International Publication No. WO 2018/022421 A1 (Patent Document 2), Japanese Unexamined Patent Publication No. JP-A-2020-121059 (Patent Document 3), Japanese Unexamined Patent Publication No. JP-A-2010-213800 (Patent Document 4), and the like.

### BACKGROUND ART DOCUMENTS

### PATENT DOCUMENTS

Patent Document 1: JP-A-2017-536158
Patent Document 2: WO 2018/022421 A1
Patent Document 3: JP-A-2020-121059
Patent Document 4: JP-A-2010-213800

### SUMMARY OF THE INVENTION

### PROBLEM THE INVENTION ATTEMPTS TO SOLVE

Meanwhile, Patent Document 1 and Patent Document 2 show a catheter in which operations such as bending its distal portion can be performed by applying force toward the distal or proximal end side to an operation wire fixed to its distal portion. In Patent Document 1, the operation wire is fixed to the distal portion of the catheter by welding the operation wire to a pull ring fixed to the distal portion of the catheter, or the like. In Patent Document 2, the operation wire is fixed to the distal portion of the catheter by unraveling the distal portion of the operation wire, which is made of a large number of metal strands twisted together, and bonding the unraveled distal portion of the operation wire to the catheter with an adhesive.

However, in the structure of Patent Document 1, since a metallic pull ring is arranged on the distal portion of the catheter, the distal portion of the catheter may become locally hard at the location of the pull ring, posing a risk of adversely affecting the balance of hardness (deformation rigidity) of the catheter.

Also, in the structure of Patent Document 2, it is complicated to unravel the distal portion of the thin operation wire, and it is necessary to precisely inject the adhesive into the operation lumen through which the operation wire is inserted, which increase the manufacturing time. Moreover, the diameter of the operation lumen needs to be large enough to allow injection of the adhesive, and there is a possibility that the diameter of the operation lumen may become a constraint when making the catheter smaller in diameter.

Patent Document 3 shows a catheter in which a lumen is formed by a braided tube having a braided layer. In Patent Document 3, a pull ring is fitted externally onto the tip end of the braided layer, and it is proposed that the pull ring prevents the strands constituting the braided layer from penetrating an outside resin layer and projecting to the outside.

However, in the structure of Patent Document 3, since a metallic pull ring is arranged on the distal portion of the catheter, the distal portion of the catheter may become locally hard at the location of the pull ring, posing a risk of adversely affecting the balance of hardness of the catheter. Besides, when the pull ring is swaged externally onto the braided layer by crimping, external force at the time of crimping may cause unintended deformation or the like of the distal portion of the catheter.

Furthermore, if a plurality of lumens are provided in the braided tube, the arrangement of each lumen according to its intended use or the like is important in setting the characteristics of the catheter, but such discussion has not been sufficiently held.

Patent Document 4 shows a catheter in which operations such as bending its distal portion can be performed by applying force toward the distal or proximal end side to an operation wire fixed to the distal portion. Patent Document 4 shows a catheter assembly in which a controller is provided on the proximal end side of the catheter to control force input to the operation wire, and the proximal portion of the catheter is inserted into the controller.

However, with the structure of Patent Document 4, there is a possibility that the catheter and the controller may move relative to each other when the force is applied to the operation wire. For example, if the catheter is provided so as to penetrate the controller, it is conceivable that the catheter and the controller are positioned on the proximal end side of the controller. However, even in that case, misalignment of the catheter and the controller could occur due to the catheter being deformed to curve in the controller when the operation wire is pulled toward the proximal end side.

Patent Document 4 shows a catheter including a suction lumen. The catheter of Patent Document 4 can be deformed to curve by the operation wire inserted through an operation lumen, and the direction of opening of the suction lumen can be suitably changed to the lateral side accordingly. Besides, a hub or the like is connected to the proximal end side of a catheter main body, which includes the suction lumen and the operation lumen. The hub or the like may be directly connected to the catheter main body, or may be indirectly connected via a connection tube for connection, for example.

However, in the structure of Patent Document 4, which includes the suction lumen and the operation lumen, when attempting to connect the proximal end of the catheter main body and the hub or the like via a connection tube, the suction lumen and the operation lumen may short-circuit each other on the proximal end side. Specifically, the connection tube is fastened to the catheter main body by its distal portion being externally fitted onto the catheter main body while being abutted against to the proximal end face of the catheter main body. Here, due to dimensional errors in the connection tube or the catheter main body, etc., the proximal end opening of the operation lumen may not be completely covered by the connection tube. In such a case, a short circuit between the suction lumen and the operation lumen could occur, posing a risk of decrease in suction force of the suction lumen.

It is an object of the present invention to provide a catheter or a catheter assembly with a novel structure which is able to solve at least one of the above-mentioned problems of the catheter or the catheter assembly.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a catheter comprising: a main lumen; an operation lumen; an operation wire inserted through the operation lumen, the operation wire being fixed on a distal end side such that the catheter is operable; and a distal end tube fastened in a butted state to a periphery of a distal end opening of the main lumen, wherein the operation wire extending out of a distal end opening of the operation lumen is adhered to an outer circumferential surface of the distal end tube.

According to the catheter structured following the present preferred embodiment, the distal end tube is fastened in the butted state to the periphery of the distal end opening of the main lumen, and the operation wire is adhered to the outer circumferential surface of the distal end tube. With this configuration, the portion where the operation wire is fastened does not become extremely hard, unlike the case of providing a metal pull ring, and the hardness of the distal portion of the catheter can be well balanced. Therefore, for example, when the catheter is deformed to curve by the operation wire, the distal portion of the catheter can be deformed smoothly in its entirety, thereby preventing localized pressing of the catheter against the somatic lumen due to strain deformation.

A second preferred embodiment provides the catheter according to the first preferred embodiment, wherein a tip end part of the operation wire adhered to the outer circumferential surface of the distal end tube has a bent shape.

According to the catheter structured following the present preferred embodiment, improvement in strength and reliability of the adhesion of the tip end part of the operation wire to the distal end tube can be achieved. The specific bent shape of the tip end part of the operation wire is not limited.

A third preferred embodiment provides a catheter comprising a braided tube forming a lumen, the braided tube comprising: a braided layer; an inside resin layer covering a radially inner side of the braided layer; and an outside resin layer covering a radially outer side of the braided layer, wherein at a distal end of the braided layer, the braided layer is not covered by the outside resin layer while a heat shrinkable tube is directly overlapped and fitted on the braided layer, and a covering layer made of resin covers the heat shrinkable tube.

According to the catheter structured following the present preferred embodiment, the heat shrinkable tube, which has been shrunk by heating, is fitted in a state of close contact on the distal end of the braided layer by being directly overlapped thereon. Thus, the distal end of the braided layer is effectively covered by the heat shrinkable tube throughout its entirety, and the strands constituting the braided layer are prevented from jutting out. Moreover, since the heat shrinkable tube is covered by the covering layer, the strands are prevented from jutting out by the covering layer as well. Additionally, the heat shrinkable tube is provided separately from the outside resin layer, and is covered by the covering tube. This reduces restrictions on the material due to insertability into the somatic lumen, corrosion resistance, and the like, making it easy to select a material that has excellent performance in preventing the strands from jutting out.

A fourth preferred embodiment provides the catheter according to the third preferred embodiment, wherein the covering layer comprises a tubular part on a base end side of a distal end tip.

According to the catheter structured following the present preferred embodiment, the covering layer comprises a portion of the distal end tip, thereby preventing the strands from jutting out with a small number of parts.

A fifth preferred embodiment provides the catheter according to the third or fourth preferred embodiment, wherein the braided tube comprises a main lumen and an operation lumen, and the braided layer surrounds both the main lumen and the operation lumen.

According to the catheter structured following the present preferred embodiment, the braided layer reduces the deformation of the main lumen, thereby achieving a main lumen with excellent shape stability. By the operation lumen being surrounded by the braided layer, when external force is applied to the operation wire inserted through the operation lumen, it is possible to avoid troubles such as the tube being torn by pressing of the operation wire and exposing the operation wire.

A sixth preferred embodiment provides the catheter according to the fifth preferred embodiment, further comprising: a distal end tube fastened in a butted state to a periphery of a distal end opening of the main lumen; and an operation wire extending out of a distal end opening of the operation lumen and adhered to an outer circumferential surface of the distal end tube, wherein a portion of the distal end tube where the operation wire is adhered is covered by the heat shrinkable tube.

According to the catheter structured following the present preferred embodiment, the distal end tube is fastened in the butted state to the periphery of the distal end opening of the main lumen, and the operation wire is adhered to the outer circumferential surface of the distal end tube. With this configuration, the portion where the operation wire is fastened does not become extremely hard, unlike the case of providing a metal pull ring, and the hardness of the distal portion of the catheter can be well balanced. Therefore, for example, when the catheter is deformed to curve by the operation wire, the distal portion of the catheter can be deformed smoothly in its entirety, thereby preventing localized pressing of the catheter against the somatic lumen due to strain deformation.

Furthermore, the portion where the operation wire is adhered to the distal end tube is covered by the heat shrinkable tube. This makes it possible to more advantageously improve reliability and adhesive strength of the adhered structure between the operation wire and the distal end tube.

A seventh preferred embodiment provides a catheter assembly comprising: a catheter comprising a main lumen and an operation lumen; and a controller attached to a proximal end side of the catheter, the controller comprising a housing and an operating part, the operating part applying operating force to a proximal end side of an operation wire inserted through the operation lumen, wherein the housing of the controller includes a positioning mechanism positioning the catheter with respect to the housing in a length direction in a portion where the catheter is retracted into the housing.

According to the catheter assembly structured following the present preferred embodiment, when the operating force is applied to the operation wire by the operating part, the positioning mechanism prevents the catheter from moving in the length direction with respect to the controller by the operating force. This makes it possible to efficiently transmit the operating force to the distal end side of the catheter to effectively induce bending deformation of the catheter.

An eighth preferred embodiment provides the catheter assembly according to the seventh preferred embodiment, wherein the positioning mechanism comprises: a stopper protrusion projecting from an outer circumferential surface of the catheter; and a stopper abutting part provided to the housing of the controller, the stopper abutting part abutting against the stopper protrusion to define a moving end of the catheter.

According to the catheter structured following the present preferred embodiment, the positioning mechanism for positioning the catheter in the length direction with respect to the controller can be achieved with a simple structure by abutment between the stopper protrusion and the stopper abutting part.

A ninth preferred embodiment provides a catheter comprising: a catheter main body comprising a suction lumen and an operation lumen, the operation lumen allowing an operation wire to be inserted; and a connection tube for connection with another member such as a connection hub, the connection tube being fastened to a proximal end side of the catheter main body, wherein a sealing tube is fastened to a proximal end face of the catheter main body by being overlapped in a butted state on a periphery of an opening of the suction lumen such that a proximal end opening of the operation lumen is shut off from the suction lumen by the sealing tube, while the suction lumen communicates with the connection tube through an inner hole of the sealing tube, a covering tube is fitted externally astride the catheter main body and the sealing tube and covers outer circumferential surfaces of the catheter main body and the sealing tube, and the connection tube comprises the sealing tube and the covering tube.

According to the catheter structured following the present preferred embodiment, the sealing tube is overlapped on and fastened to the proximal end side of the catheter main body where the operation lumen opens. This prevents the suction lumen and the operation lumen from short-circuiting each other on the proximal end side and allows suction force to act on the suction lumen efficiently. Besides, the covering tube is fastened externally astride the catheter main body and the sealing tube, thereby improving fastening strength of the connection tube with respect to the catheter main body. The sealing tube covering the proximal opening of the operation lumen is provided separately from the covering tube fitted externally onto the catheter main body. Thus, even if the connection tube that is externally fastened onto the catheter main body is adopted, it is possible to prevent insufficient blockage of the base end opening of the operation lumen due to, for example, an error in the outer diameter dimension of the catheter main body or the like.

A tenth preferred embodiment provides the catheter according to the ninth preferred embodiment, wherein the proximal end opening of the operation lumen is covered by the sealing tube, and the operation wire is taken out of the operation lumen at a location on a tip end side with respect to the covering tube.

According to the catheter structured following the present preferred embodiment, there is no need to form a hole or the like in the covering tube to take out the operation wire, and the covering tube is fastened approximately uniformly about the entire circumference.

An eleventh preferred embodiment provides the catheter according to the ninth preferred embodiment, wherein the operation wire extending out of the proximal end opening of the operation lumen passes through the sealing tube and the covering tube and is taken out of an outer circumferential surface of the covering tube.

According to the catheter structured following the present preferred embodiment, there is no need to form a hole or the like in the catheter main body to take out the operation wire. It is possible to prevent a short circuit between the suction lumen and the operation lumen by the sealing tube, while taking out the base end side of the operation wire through the sealing tube and the covering tube to the outside of the catheter.

A twelfth preferred embodiment provides the catheter according to the eleventh preferred embodiment, wherein the catheter main body further comprises a braided layer provided in an embedded state and surrounding both the suction lumen and the operation lumen, and the suction lumen and the operation lumen are provided in an internal region surrounded by the braided layer.

According to the catheter structured following the present preferred embodiment, by adopting the braided tube as the catheter main body, required characteristics such as pressure resistance and kink resistance are achieved to a high degree, while it is not necessary to provide a through hole in the braided tube to take out the operation wire. This makes it possible to easily realize a structure in which the base end side of the operation wire is taken out to the outside of the catheter.

A thirteenth preferred embodiment provides a catheter comprising a braided tube, wherein a suction lumen subjected to negative pressure and an operation lumen allowing an operation wire to be inserted are provided and extend in parallel, a braided layer is provided in an embedded state and surrounds both the suction lumen and the operation lumen, the suction lumen and the operation lumen being provided in an internal region surrounded by the braided layer, and a guide wire lumen configured to allow a guide wire to be inserted is provided in an external region outside the internal region surrounded by the braided layer.

According to the catheter structured following the present preferred embodiment, the suction lumen is provided in the internal region surrounded by the braided layer, and the shape stability of the suction lumen is enhanced by reinforcing action of the braided layer. When the suction force (the negative pressure) acts on the suction lumen, pressure compensation action due to the deformation of the suction lumen is reduced, thereby efficiently transmitting the suction force to the distal end.

Besides, the operation lumen is provided in the internal region surrounded by the braided layer. Thus, when external force is applied to the operation wire inserted through the operation lumen, even if the tube is torn by pressing of the operation wire, it is possible to prevent the operation wire from being exposed to the outside.

The guide wire lumen is provided in the external region outside the internal region surrounded by the braided layer. This makes it also possible to prevent characteristics such as bending rigidity of the tube from excessively increasing.

A fourteenth preferred embodiment provides the catheter according to the thirteenth preferred embodiment, wherein a portion forming the suction lumen and the operation lumen comprises the braided tube having a circular outer circumferential surface, the braided tube comprising: an inside resin layer covering a radially inner side of the braided layer; and an outside resin layer covering a radially outer side of the braided layer, and a region forming the guide wire lumen projects from the circular outer circumferential surface of the braided tube.

According to the catheter structured following the present preferred embodiment, by making the braided layer circular in cross section, bending deformation characteristics are likely to be approximately uniform about the entire circumference, and it is possible to induce bending deformation by the operation wire in any radial direction. Besides, the region forming the guide wire lumen, where large forces such as the suction force and the operating force do not act, is provided so as to project outside the braided layer, thereby keeping the diameter dimension of the braided layer small and preventing excessive increase in bending rigidity by the braided layer.

A fifteenth preferred embodiment provides a catheter comprising: a catheter main body comprising a suction lumen and an operation lumen, the operation lumen allowing an operation wire to be inserted; and a connection tube for connection with another member such as a connection hub, the connection tube being fastened to a proximal end side of the catheter main body, wherein a distal end part of the connection tube is fastened to a proximal end part of the catheter main body by being overlapped in a butted state at a location off an opening of the operation lumen, the suction lumen of the catheter main body communicates with the connection tube, and the operation lumen of the catheter main body is open on a base end side of the catheter main body.

According to the catheter structured following the present preferred embodiment, by opening the suction lumen directly to the outside of the catheter on the proximal end side, the operation wire can be easily taken out of the catheter with a simple structure. Moreover, the connection tube is fastened to the proximal end side of the catheter main body in the butted state at the location off the opening of the action lumen, and the suction lumen communicates with the connection tube, thereby enabling suction treatments through the suction lumen.

A sixteenth preferred embodiment provides the catheter according to the fifteenth preferred embodiment, wherein at the proximal end part of the catheter main body, a proximal end opening portion of the suction lumen has an obliquely cut shape such that the proximal end opening portion of the suction lumen is shorter than a proximal end opening portion of the operation lumen, and a distal end opening portion of the connection tube is fastened by being overlapped in the butted state via an obliquely cut shape corresponding to that of the proximal end opening portion of the suction lumen.

According to the catheter structured following the present preferred embodiment, in the portion where the connection tube is connected to the catheter main body, the overlapping area or the adhesive area of the two members can be more largely ensured, thereby making it possible to fasten the two members more firmly with high reliability.

### EFFECT OF THE INVENTION

According to the present invention, it is possible to solve at least one of the above-mentioned problems of the conventional catheter or catheter assembly.

### BRIEF DESCRIPTION OF THE DRAWINGS

FIG. 1 is a vertical cross-sectional view of a suction catheter according to a first practical embodiment of the present invention.
FIG. 2 is an enlarged cross-sectional view taken along line 2-2 of FIG. 1.
FIG. 3 is a diagrammatic view showing a manufacturing process of the suction catheter shown in FIG. 1, which is suitable for explaining a step of fastening an operation wire to a distal end tube.
FIG. 4 is a diagrammatic view showing a manufacturing process of the suction catheter shown in FIG. 1, which is suitable for explaining a step of attaching a heat shrinkable tube.
FIG. 5 is a diagrammatic view showing a manufacturing process of the suction catheter shown in FIG. 1, which is suitable for explaining a step of attaching a distal end tip.
FIG. 6 is a perspective view of a catheter assembly in which a controller is attached to a proximal end of the suction catheter shown in FIG. 1.
FIG. 7 is a view of a positioning mechanism inside the controller in the catheter assembly shown in FIG. 6.
FIG. 8 is a view suitable for explaining a suction catheter according to a second practical embodiment of the present invention, corresponding to a plan view showing a portion where an operation wire is adhered to a distal end tube, with a distal end tip and a heat shrinkable tube omitted.
FIG. 9 is an enlarged vertical cross-sectional view suitable for explaining a suction catheter according to a third practical embodiment of the present invention, showing a portion where a sealing tube and a covering tube are connected to a proximal end side of a catheter main body.
FIG. 10 is an enlarged side view suitable for explaining a suction catheter according to a fourth practical embodiment of the present invention, showing a portion where a connection tube is connected to a proximal end side of a catheter main body.
FIG. 11 is an enlarged vertical cross-sectional view suitable for explaining a principal part of the suction catheter shown in FIG. 10.
FIG. 12 is a cross-sectional view taken along line 12-12 of FIG. 11.
FIG. 13 is a cross-sectional view taken along line 13-13 of FIG. 11.

### EMBODIMENTS FOR CARRYING OUT THE INVENTION

Hereinafter, practical embodiments of the present invention will be described with reference to the drawings.

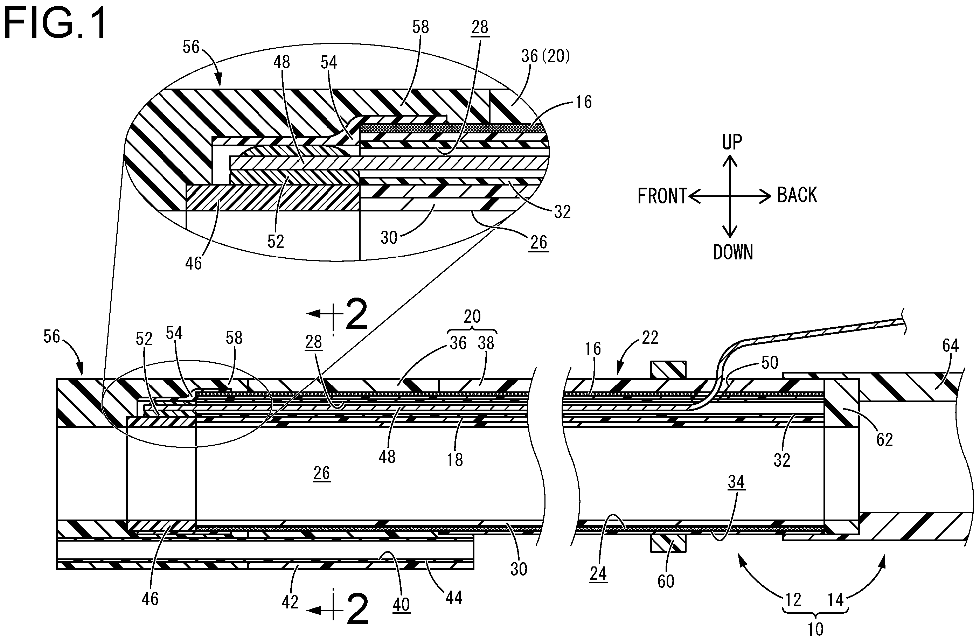

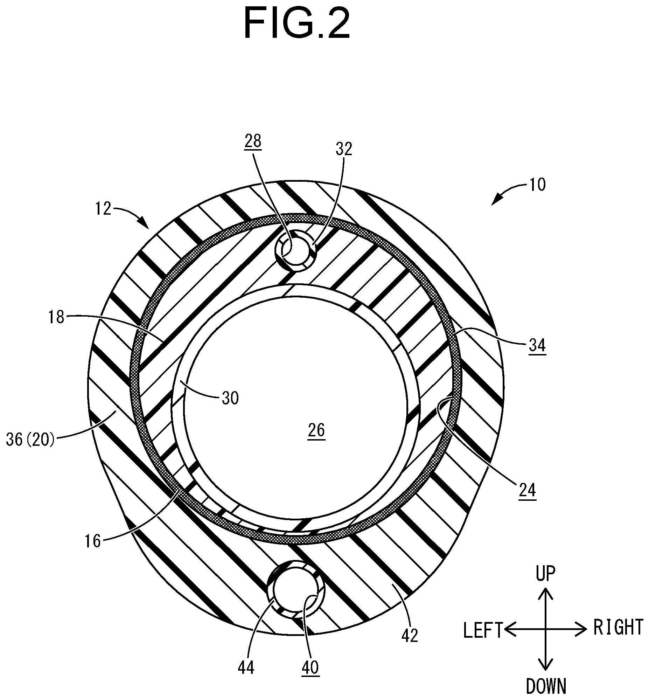

FIGS. 1 and 2 depict a catheter 10 according to a first practical embodiment of the present invention. The catheter 10 is a suction catheter used for thrombus suction therapy to suction thrombi or the like in a blood vessel. The catheter 10 has a structure in which a connection tube 14 is fastened to the proximal end side of a catheter main body 12. In the following description, as a general rule, the vertical direction refers to the vertical direction in FIG. 1, the front-back direction refers to the left-right direction in FIG. 1, which coincides with the axial direction, and the left-right direction refers to the left-right direction in FIG. 2. Besides, as a general rule, the tip end side refers to the distal end side, and the base end side refers to the proximal end side.

The catheter main body 12 includes a braided tube 22 having a braided layer 16, an inside resin layer 18 covering the radially inner side of the braided layer 16, and an outside resin layer 20 covering the radially outer side of the braided layer 16.

The braided layer 16 has a structure in which a large number of braided metal strands (braided wires) such as medical stainless steel are braided together. The braided layer 16 has a tubular shape with an approximately circular cross section overall, and the deformation characteristics (bending rigidity) during bending deformation are approximately constant about the entire circumference. However, the braided layer 16 may have a cross-sectional shape other than a circle, such as an oval, for example, or the cross-sectional shape may vary in the axial direction.

The inside resin layer 18 is provided in an internal region 24 surrounded by the braided layer 16. A suction lumen 26 serving as a main lumen and an operation lumen 28, which extend in the axial direction (the length direction) of the braided tube 22, are formed in the internal region 24. The braided layer 16 is arranged on the radially outer side so as to surround both the suction lumen 26 and the operation lumen 28. The suction lumen 26 and the operation lumen 28 extend approximately parallel to each other. In the present practical embodiment, the suction lumen 26 has a larger diameter and has a larger cross-sectional area than those of the operation lumen 28. The suction lumen 26 is provided at a position eccentric downward with respect to the center of the cross section of the braided layer 16, and the operation lumen 28 is provided above the suction lumen 26. Both the suction lumen 26 and the operation lumen 28 are located in the internal region 24 surrounded by the braided layer 16. The inner circumferential surface of the suction lumen 26 is covered by a coating layer 30 to, for example, improve corrosion resistance to blood contact, reduce flow resistance of blood, and the like. The inner circumferential surface of the operation lumen 28 is covered by a coating layer 32 to, for example, reduce sliding resistance of an operation wire 48 (described later), and the like. The material of the coating layers 30, 32 is not particularly limited, but is, for example, a fluororesin layer. The coating layer 30 of the suction lumen 26 and the coating layer 32 of the operation lumen 28 may be made of the same material, or they may be made of different materials from each other to meet the respective performance requirements to a higher degree.

The outside resin layer 20 is provided in an external region 34, which is the radially outer side of the braided layer 16 outside the internal region 24. The outside resin layer 20 may be integral in its entirety in the axial direction, but in the present practical embodiment, the outside resin layer 20 is composed of a distal resin layer 36 and a proximal resin layer 38, which are made of mutually different materials.

The distal resin layer 36 includes a guide wire lumen 40. That is, in the lower part of the distal resin layer 36, a projecting part 42 is provided with a larger projecting height from the braided layer 16, and the guide wire lumen 40 is provided so as to penetrate the projecting part 42 in the axial direction. The inner circumferential surface of the guide wire lumen 40 is covered by a coating layer 44 to, for example, improve corrosion resistance, reduce resistance during insertion of a guide wire, and the like. The coating layer 44 of the guide wire lumen 40 may be made of the same material as the coating layer 30 of the suction lumen 26 or the coating layer 32 of the operation lumen 28, or may be made of a different material from those coating layers 30, 32.

In this way, regarding the braided tube 22 including the outside resin layer 20, the portion forming the suction lumen 26 and the operation lumen 28 has an approximately circular outer circumferential surface, and the guide wire lumen 40 is formed in the projecting part 42 projecting from the outer circumferential surface.

In the present practical embodiment, the distal resin layer 36 and the proximal resin layer 38 are butted against and welded to each other in the axial direction at the portion circumferentially off the projecting part 42. Meanwhile, at the lower part where the projecting part 42 is provided, the distal resin layer 36 projects to the base end side, and the projecting base end portion of the distal resin layer 36 is overlapped on and welded to the outer circumferential surface of the tip end portion of the proximal resin layer 38. This configuration makes it possible to increase fastening strength between the distal resin layer 36 and the proximal resin layer 38, compared to the case where the distal resin layer 36 and the proximal resin layer 38 are fastened in a butted state about the entire circumference.

In braided tube 22, the tip end of outside resin layer 20 does not reach the tip end of the braided layer 16, and the braided layer 16 is exposed without being covered by the outside resin layer 20 on the tip end side with respect to the outside resin layer 20.

A distal end tube 46 is provided on the tip end side of the inside resin layer 18. The distal end tube 46 has a round tubular shape. The distal end tube 46 is fastened in a butted state to the tip end face of the inside resin layer 18 at the periphery of the tip end opening of the suction lumen 26, and projects to the tip end side. The distal end tube 46 is made of a flexible resin elastomer, for example. The coating layer 30 provided on the inner circumferential surface of the inside resin layer 18 may extend out to the inner circumferential surface of the distal end tube 46. **In** such a case, it is desirable that the inner diameter of the coating layer 30 be approximately the same over both the inside resin layer 18 and the distal end tube 46, for example, by suitably making the inner diameter of the distal end tube 46 approximately the same as that of the inside resin layer 18, or the like.

An operation wire 48 inserted through the operation lumen 28 is adhered to the distal end tube 46. The operation wire 48 is formed, for example, of a single metal strand or a large number of metal strands twisted together, and is inserted through the operation lumen 28 movably in the axial direction. The base end part of the operation wire 48 extends out to the outside through a port 50 opening to the lateral side on the base end side of the operation lumen 28. The tip end part of the operation wire 48 extends out of the operation lumen 28 to the tip end side, and is adhered to the outer circumferential surface of the distal end tube 46 with an adhesive 52. In the present practical embodiment, since the operation lumen 28 is located above the distal end tube 46, the operation wire 48 is overlapped on the upper surface of the distal end tube 46 and fixed to the distal end tube 46 by the adhesive 52. By the operation wire 48 being pulled toward the base end side, the catheter main body 12 comprising the braided tube 22 is deformed to curve, and the orientation of the tip end of the catheter main body 12 can be manipulated by operation of the operation wire 48.

The adhered site between the distal end tube 46 and the operation wire 48 is covered by a heat shrinkable tube 54. The heat shrinkable tube 54 is made of resin and decreases in diameter when heated. In the initial state before heating, the heat shrinkable tube 54 is larger in diameter than the connected portion between the distal end tube 46 and the operation wire 48, and is placed externally about the connected portion. The heat shrinkable tube 54 placed externally about the connected portion between the distal end tube 46 and the operation wire 48 is shrunk by heating and comes into close contact with the said connected portion.

The base end part of the heat shrinkable tube 54 reaches the outer circumference of the braided layer 16, which is exposed on the tip end side with respect to the outside resin layer 20. The heat shrinkable tube 54, which has been shrunk by heating, is directly overlapped and fitted on the tip end part of the braided layer 16 in a state of close contact.

The tip end part of the catheter main body 12 comprises a distal end tip 56. The distal end tip 56 is made of resin, for example, and has a tubular shape overall, including a lumen comprising the tip end part of the suction lumen 26. The distal end tip 56 preferably has deformation rigidity higher than that of the braided tube 22. The tip end part of the distal end tip 56 is fastened to the tip end face of the distal end tube 46, and is located on the tip end side with respect to the distal end tube 46. The base end portion of the distal end tip 56 comprises a tubular part 58 serving as a covering layer that extends out to the base end side with respect to the heat shrinkable tube 54. The tubular part 58 is overlapped on the outer circumferential surface of the heat shrinkable tube 54 and externally covers the entire heat shrinkable tube 54. Besides, the base end part of the tubular part 58, which is located beyond the heat shrinkable tube 54 to the base end side, is overlapped directly on the outer circumferential surface of the braided layer 16, and the base end face of the tubular part 58 is axially butted against and welded to the distal resin layer 36. The distal end tip 56 is welded to each of the distal end tube 46, the heat shrinkable tube 54, and the braided layer 16.

The tip end portion of the guide wire lumen 40 is provided in the lower part of the distal end tip 56, and is axially continuous with the base end portion of the guide wire lumen 40 provided in the outside resin layer 20. In the present practical embodiment, the tip end opening of the guide wire lumen 40 is set at the same location as the tip end opening of the suction lumen 26 in the axial direction, while the base end opening is located on the tip end side with respect to the base end opening of the suction lumen 26 in the axial direction. Thus, the catheter 10 of the present practical embodiment is a rapid exchange (RX) type catheter. A guide wire (not shown) is configured to be inserted through the guide wire lumen 40, which is formed cooperatively by the outside resin layer 20 and the distal end tip 56.

A stopper protrusion 60 is attached to the base end portion of the catheter main body 12. The stopper protrusion 60 is made of resin, metal, or the like, for example, and projects radially outward from the outside resin layer 20 of the catheter main body 12. The stopper protrusion 60 of the present practical embodiment has an annular shape and is provided on the outer circumferential surface of the outside resin layer 20 about the entire circumference. However, the stopper protrusion 60 may be provided, for example, partially on the circumference on the outer circumferential surface of the outside resin layer 20. The method of fixing the stopper protrusion 60 to the outside resin layer 20 is not particularly limited, but for example, if the stopper protrusion 60 is made of resin, it can be fixed by welding, and if the stopper protrusion 60 is made of metal, it can be fixed by swaging.

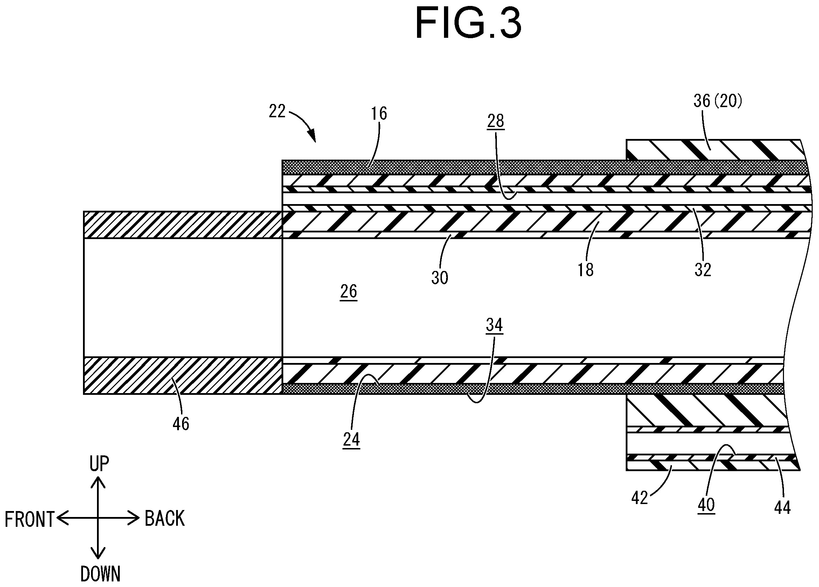

The catheter main body 12 of the above construction can be obtained, for example, in the following manner. Specifically, first, the braided tube 22 including the braided layer 16, to which the inside resin layer 18 and the outside resin layer 20 are fastened, is prepared. Here, the stopper protrusion 60 may be provided in advance on the prepared braided tube 22, or alternatively, for example, a step for providing the stopper protrusion 60 may be set after the formation of the distal end tip 56, which will be described later. Next, as shown in FIG. 3, at the tip end part of the braided tube 22, the outside resin layer 20 is removed to expose the braided layer 16. Additionally, the distal end tube 46 is butted against and joined to the tip end face of the braided tube 22.

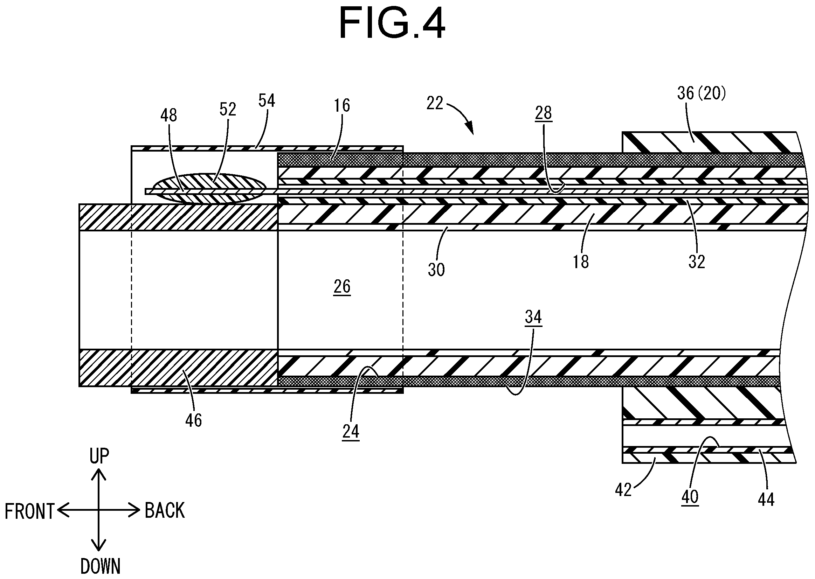

Subsequently, as shown in FIG. 4, the operation wire 48 is inserted through the operation lumen 28 of the braided tube 22, and the tip end part of the operation wire 48 is adhered and fixed to the distal end tube 46 with the adhesive 52. Besides, the heat shrinkable tube 54 is placed externally so as to straddle the exposed tip end part of the braided layer 16 and the adhered site between the distal end tube 46 and the operation wire 48.

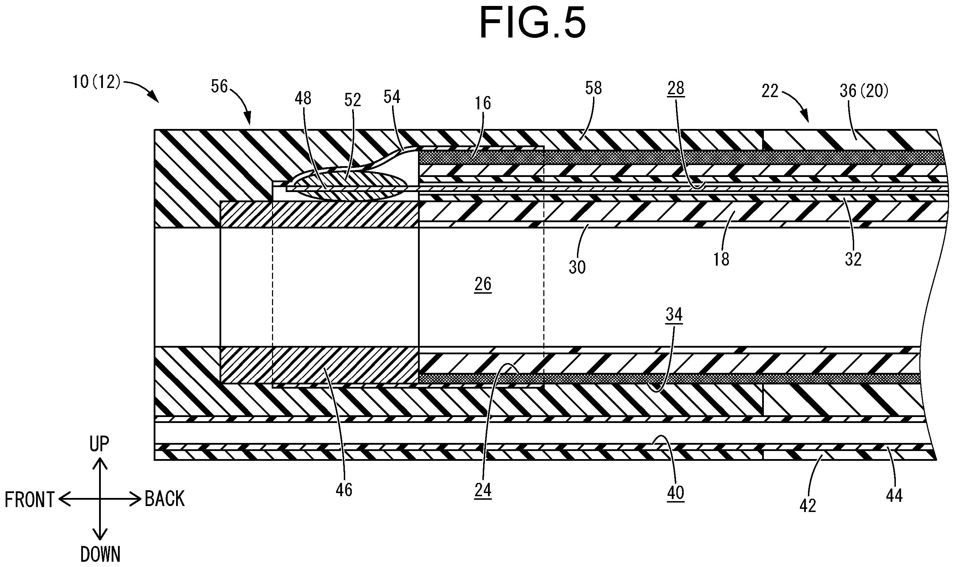

Then, the heat shrinkable tube 54 is heated and shrunk, such that the heat shrinkable tube 54 is brought into close contact with the tip end part of the braided layer 16 and the fixed site between the distal end tube 46 and the operation wire 48, as shown in FIG. 5. By so doing, the fixation of the distal end tube 46 and the operation wire 48 is reinforced by the heat shrinkable tube 54, and the tip end of the metal strand is covered by the heat shrinkable tube 54 at the tip end part of the braided layer 16. The heat shrinkable tube 54 is overlapped on the tip end part of the braided tube 22 from which the outside resin layer 20 has been removed, and is directly overlapped to be in close contact with the braided layer 16.

After heating and diameter constriction of the heat shrinkable tube 54, the distal end tip 56 is formed to obtain the catheter main body 12. By so doing, the distal end tip 56 is provided so as to project from the tip end side of the distal end tube 46, and the radially outer side of the heat shrinkable tube 54 is covered by the tubular part 58, which is the base end portion of the distal end tip 56. Accordingly, the fixing strength of each component constituting the tip end part of the catheter main body 12 is enhanced by the distal end tip 56.

In the catheter 10 having the catheter main body 12 constructed as above, the tip end part of the operation wire 48 is adhered to the flexible distal end tube 46 which projects to the tip end side of the braided tube 22. With this configuration, it is possible to effectively fasten the tip end part of the operation wire 48 to the tip end part of the catheter main body 12 while preventing the tip end part of the catheter main body 12 from becoming excessively hard due to the fixing structure of the operation wire 48. This makes it possible to achieve ease of insertion into the somatic lumen and minimally invasive property owing to good hardness balance of the catheter main body 12.

The portion of the operation wire 48 adhered to the catheter main body 12 is covered by the heat shrinkable tube 54 in a state of close contact. This arrangement achieves improvement in fastening strength of the operation wire 48 to the catheter main body 12 owing to supplementary fastening by the heat shrinkable tube 54. Besides, problems such as peeling of the portion adhered by the adhesive 52 are unlikely to occur.

The heat shrinkable tube 54 is overlapped and fitted directly on the tip end part of the braided layer 16 exposed radially outward, and the tip end of the braided layer 16 is protected by the heat shrinkable tube 54. Moreover, the heat shrinkable tube 54 is covered by the tubular part 58 of the distal end tip 56. This makes it possible to prevent the tip ends of the metal strands, which constitute the braided layer 16, from damaging the outside resin layer 20 and being exposed radially outward when the catheter 10 curves. In particular, if the catheter main body 12 can be curved by inputting force to the operation wire 48, a large bending force acts on the braided layer 16, and the metal strands are likely to be exposed radially outward. However, the catheter 10 of the present practical embodiment, in which the tip end part of the braided layer 16 is covered by the heat shrinkable tube 54 and the tubular part 58 of the distal end tip 56, prevents exposure of the metal strands radially outward. Moreover, the covering layer covering the heat shrinkable tube 54 is constituted by the tubular part 58, which is a part of the distal end tip 56. Thus, the number of parts can be reduced compared to the case where the covering layer is provided as a separate member.

Additionally, the suction lumen 26 is provided in the internal region 24 of the braided layer 16. Thus, when negative pressure is applied to the suction lumen 26 to suction thrombi or the like, deformation of the suction lumen 26 due to action of the negative pressure is suppressed by reinforcing action of the braided layer 16. Therefore, pressure compensation action due to the deformation of the suction lumen 26 is reduced, thereby efficiently transmitting the negative pressure to the tip end opening of the suction lumen 26.

The operation lumen 28 through which the operation wire 48 is inserted is provided in the internal region 24 of the braided layer 16. Thus, even if the internal region 24 is torn due to contact of the operation wire 48 subjected to external force, the braided layer 16 prevents exposure of the operation wire 48 to the outside, thereby avoiding troubles such as contact of the operation wire 48 to the somatic lumen.

The guide wire lumen 40 is provided in the external region 34 without being surrounded by the braided layer 16. This prevents the braided layer 16 from being excessively large in diameter and avoids excessive increase in moment of inertia of area of the braided layer 16 with respect to bending. This minimizes the bending rigidity of the catheter main body 12, thereby achieving good insertability of the catheter main body 12 into the somatic lumen, excellent curve-following ability of the catheter main body 12 with respect to operating force applied by the operation wire 48, and the like. In particular, in the RX-type catheter main body 12 in which the guide wire lumen 40 is provided only in the tip end portion, since the guide wire lumen 40 is provided in the external region 34 of the braided layer 16, there is no need to provide a hole or the like in the braided layer 16 to take out the guide wire. Besides, the guide wire lumen 40, through which the guide wire is inserted, does not need to be surrounded and protected by the braided layer 16 because external forces such as suction force acting on the suction lumen 26 and operating force exerted on the operation wire 48 inserted through the operation lumen 28 are not expected to act on the guide wire lumen 40. Additionally, the braided tube 22 has an approximately circular outer shape and the guide wire lumen 40 is formed in the projecting part 42 projecting from the outer circumferential surface of the braided tube 22. Thus, it is possible to minimize increase in diameter of the catheter main body 12 comprising the braided tube 22 while providing the guide wire lumen 40 in the external region 34.

The braided layer 16 has a circular cross-section. This suppresses circumferential differences in deformation characteristics such as requiring greater operating force when curving in a particular direction, and the deformation characteristics are approximately constant about the entire circumference regardless of the curving direction.

At the base end side of the catheter main body 12 in the catheter 10, there is provided the connection tube 14 for connecting another member such as a hub (not shown) to the catheter main body 12. The connection tube 14 includes a sealing tube 62 abutted against the base end face of the catheter main body 12 and a covering tube 64 externally fitted onto the base end part of the catheter main body 12 and the sealing tube 62.

The sealing tube 62 has a tubular shape, and is arranged around the suction lumen 26. The sealing tube 62 has approximately the same outer shape as the braided tube 22, and is fastened to the periphery of the base end opening of the suction lumen 26 by being overlapped in a butted state. The sealing tube 62 is arranged so as to cover the opening of the operation lumen 28 at the base end of the braided tube 22, and the base end opening of the operation lumen 28 is airtightly blocked by the sealing tube 62.

In this way, the sealing tube 62 covering the base end opening of the operation lumen 28 is arranged so as to be butted against the base end face of the catheter main body 12. With this arrangement, the base end opening of the operation lumen 28 can be covered without any gap, thereby more reliably achieving the airtight blockage of the base end opening of the operation lumen 28. Therefore, the operation lumen 28 can be provided independently of and airtightly separated from the suction lumen 26 that is located on the radial inside of the sealing tube 62. This makes it possible to prevent the negative pressure acting on the suction lumen 26 from being reduced by a short circuit to the operation lumen 28.

The covering tube 64 is axially butted against the sealing tube 62 from the base end side. The covering tube 64 has a larger diameter than those of the sealing tube 62 and the catheter main body 12, and its outer circumferential end extends out toward the tip end side with respect to the sealing tube 62 on the radially outer side of the sealing tube 62. The tip end portion of the covering tube 64 is fitted externally astride the base end part of the catheter main body 12 and the sealing tube 62, and covers the outer circumferential surfaces of the catheter main body 12 and the sealing tube 62. Preferably, the tip end portion of the covering tube 64, which is fitted externally onto the catheter main body 12 and the sealing tube 62, is airtightly fastened to the catheter main body 12 and the sealing tube 62 by welding or other means. By the covering tube 64 being externally fastened to the catheter main body 12 and the sealing tube 62 in this way, the connection tube 14 is connected more firmly than in the case where the connection tube 14 is fastened to the catheter main body 12 only by the butted surfaces.

The tip end of the covering tube 64 is located on the base end side of the location of opening of the port 50 of the catheter main body 12, and the operation wire 48 is taken out to the outside on the tip end side with respect to the covering tube 64. Therefore, there is no need to form the port 50 so as to penetrate the covering tube 64, and the catheter main body 12 and the covering tube 64 can be fixed approximately uniformly about the entire circumference.

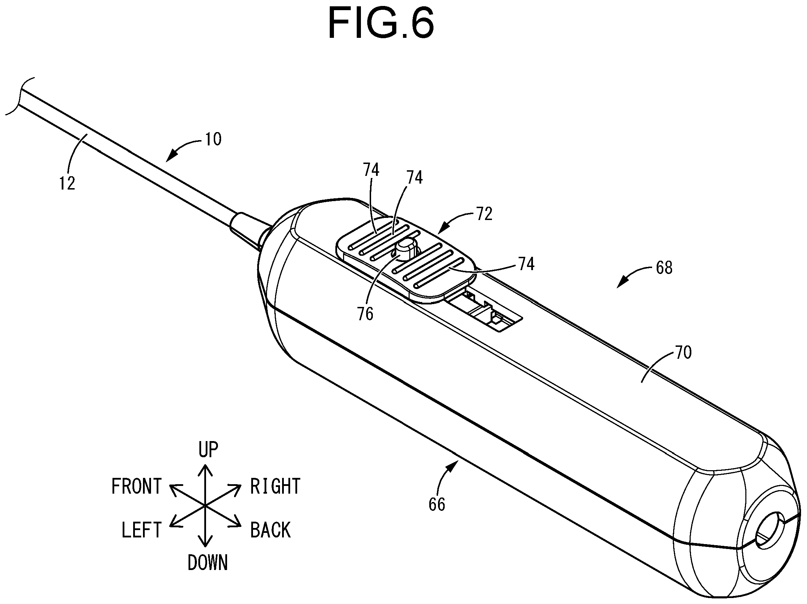

Meanwhile, as shown in FIG. 6, a controller 66 is attached to the base end side of the catheter 10 to form a catheter assembly 68. The controller 66 includes a housing 70 that houses the base end portion of the catheter 10, and an operating part 72 that is slidable with respect to the housing 70.

The housing 70 has an elongated tubular shape so as to be easily grasped and operated by a practitioner with one hand, and the catheter 10 is inserted therein in the longitudinal direction. Regarding the catheter 10, the base end part of the catheter main body 12 and the tip end part of the connection tube 14 are housed in the housing 70. The port 50 of the catheter main body 12, which is the opening from which the operation wire 48 is taken out of the operation lumen 28 to the base end side, is located within the housing 70.

The base end part of the connection tube 14 is located within the housing 70, and a hub (not shown) is connected thereto. The hub has a tubular shape, for example, and is attached to a hole penetrating the base end side wall of the housing 70 so as to interconnect the suction lumen 26, which includes the lumen of the connection tube 14, and the space outside the housing 70. A negative pressure source (a suction pump), not shown, is connected to the base end side of the hub, thereby connecting the suction lumen 26 to the negative pressure source via the hub, and allowing the negative pressure to exert on the suction lumen 26.

The operating part 72 is provided in a part of the housing 70, and is slidable with a thumb or the like while grasping the housing 70. The operating part 72 of the present practical embodiment is linearly movable relative to the housing 70 in the longitudinal direction (the axial direction of the catheter 10). In the initial state, the operating part 72 is located at the tip end of the range in which sliding displacement is possible, and is allowed to move from the initial state toward the base end side. On the upper surface of the operating part 72, a plurality of anti-slip projections 74 are provided in parallel, and extend in the left-right direction approximately orthogonally to the direction of sliding displacement. Besides, the operating part 72 is provided with a locking mechanism that prevents accidental sliding displacement. The locking mechanism is configured to be released by pushing in an unlocking button 76 projecting from the upper surface of the operating part 72. Thus, when sliding the operating part 72, the sliding displacement of the operating part 72 can be performed by inputting force to the operating part 72 toward the base end side while pushing in the unlocking button 76.

The base end part of the operation wire 48, which is taken out of the port 50 into the housing 70, is connected to the operating part 72. Therefore, when the user (the practitioner) slides the operating part 72 toward the base end side, the operation wire 48 is pulled toward the base end side, and operating force is applied to the operation wire 48. The operating force applied to the operation wire 48 is transmitted to the tip end part of the catheter main body 12 fixed to the tip end of the operation wire 48 and acts on the tip end part of the catheter main body 12 as external force toward the base end side. This allows the tip end part of the catheter main body 12 to be curved and the direction of opening of the tip end of the suction lumen 26 to be directed toward the lateral side. It may also be possible to direct the direction of opening of the tip end of the suction lumen 26 to any lateral side in the circumferential direction by twisting the controller 66 to apply circumferential torsional force on the catheter main body 12 while curving the tip end part of the catheter main body 12. The present practical embodiment shows the controller 66 that allows only pulling operation of the operation wire 48. However, for example, it would also be possible to allow the operating part 72 to move from the initial position toward the tip end side so that operating force (pushing force) toward the tip end side can be applied to the operation wire 48, whereby the tip end part of the catheter main body 12 can be curved by transmission of the pushing force.

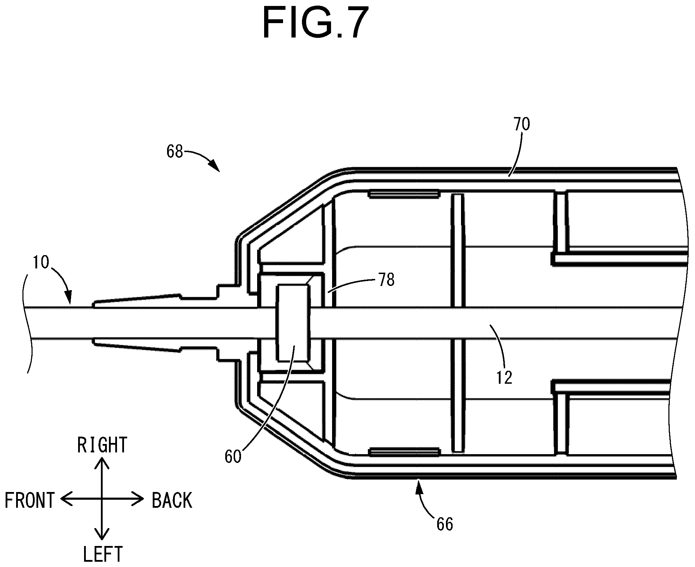

As shown in FIG. 7, the stopper protrusion 60 provided to the catheter main body 12 is housed in the tip end portion of the housing 70, which is the portion where the catheter 10 is retracted into the housing 70, and is arranged closely on the tip end side with respect to stopper abutting parts 78 in the housing 70. Each stopper abutting part 78 is integrally formed with the housing 70, and has a wall shape that extends approximately orthogonally to the axial direction of the catheter main body 12.

Each stopper abutting part 78 is made smaller in height in the vertical direction than the peripheral wall of the housing 70. Regarding the catheter 10, the base end side with respect to the stopper protrusion 60 is inserted between the stopper abutting parts 78, 78 provided on both the upper and lower sides, while the stopper protrusion 60 is made unable to pass between the stopper abutting parts 78, 78 provided on both the upper and lower sides. Accordingly, the moving end of the catheter 10 toward the base end side relative to the housing 70 is defined by abutment between the stopper protrusion 60 and the stopper abutting parts 78. In this way, in the present practical embodiment, a positioning mechanism positioning the catheter 10 with respect to the housing 70 in the front-back direction, which is the length direction, comprises the stopper protrusion 60 and the stopper abutting parts 78. The port 50 from which the operation wire 48 is taken out is located on the base end side with respect to the stopper abutting parts 78. Besides, the movement of the operation wire 48 to the base end side by sliding the operating part 72 is allowed as movement relative to the braided tube 22 and the connection tube 14 positioned with respect to the housing 70.

Since the movement of the catheter 10 toward the base end side relative to housing 70 is limited, when operating part 72 is operated to apply tensile force toward the base end side to the operation wire 48, the catheter 10 can be prevented from being retracted into the housing 70 by the operating force transmitted from the operation wire 48.

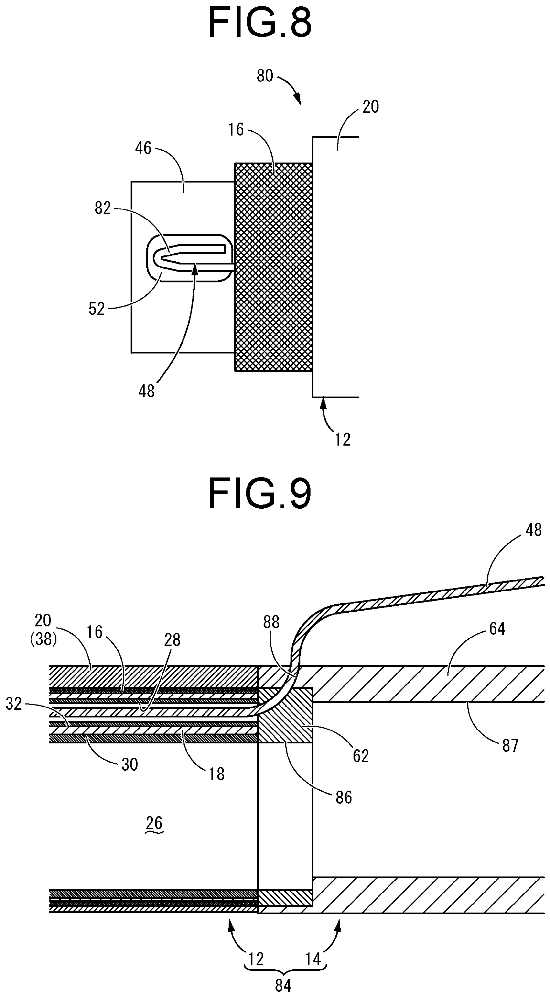

FIG. 8 shows a catheter 80 according to a second practical embodiment of the present invention. In each of the following practical embodiments, the parts and components like those in the catheter 10 of the first practical embodiment shall be designated by like reference numerals, and the corresponding parts and components will not be discussed in detail.

The catheter 80 shown in FIG. 8 is a suction catheter similar to that of the first practical embodiment. Regarding the operation wire 48 arranged penetrating through the operation lumen 28, the catheter 80 illustrates a structure different from that of the first practical embodiment with respect to the portion of the distal end adhered to the distal end tube 46. FIG. 8 is an enlarged plan view showing the distal end portion of the catheter 80, which is a principal part of the present practical embodiment (corresponding to a top view of FIG. 1 in the first practical embodiment), showing the portion of the operation wire 48 adhered to the distal end tube 46 excluding the distal end tip 56 and the heat shrinkable tube 54.

That is, in the present practical embodiment, the tip end portion of the operation wire 48 comprises a bent part 82 having a bent shape at the portion projecting from the operation lumen 28 of the catheter main body 12. In the present practical embodiment in particular, the bent part 82 has a folded shape whose extreme tip is folded in a U shape or a V shape so that the wire tip end extends in the opposite direction toward the near side of the user for a predetermined length.

The bent part 82 having the folded shape is overlapped in its entirety on the outer circumferential surface of the distal end tube 46. The bent part 82 on the distal end tube 46 is adhered to the distal end tube 46 by the adhesive 52 approximately in its entirety, including the folded portion at the extreme tip.

By adopting the operation wire 48 having the said bent part 82 at the tip end part and adhering the bent part 82 to the distal end tube 46, the adhesive area or the adhesive length of the operation wire 48 can be increased to improve adhesive strength, adhesive reliability, and the like. Additionally, the bent part 82 also exhibits anchoring action by mechanically catching on the adhesive or the like, thereby further improving the adhesive strength of the operation wire 48 to the distal end tube 46.

FIG. 9 shows a catheter 84 according to a third practical embodiment of the present invention. The catheter 84 is a suction catheter similar to that of the first practical embodiment. Regarding the operation wire 48 arranged penetrating through the operation lumen 28, the catheter 84 illustrates an embodiment different from that of the first practical embodiment with respect to the structure for taking the wire out of the catheter. FIG. 9 is an enlarged vertical cross-sectional view showing the portion of the operation wire 48 to be taken out of the catheter, which is a principal part of the present practical embodiment, and corresponds to the right side portion of FIG. 1 in the first practical embodiment.

That is, in the present practical embodiment, at the proximal end part of the catheter main body 12, a ring-shaped or tubular sealing tube 62 is fastened by welding, adhering, or the like with their end faces overlapped on each other in a butted state in the axial direction, as in the first practical embodiment. An inner hole 86 of the sealing tube 62 communicates with the suction lumen 26 of the catheter main body 12.

As in the first practical embodiment, the tip end opening of the covering tube 64 is externally connected to the sealing tube 62. Accordingly, the suction lumen 26 of the catheter main body 12 communicates with an inner hole 87 of the covering tube 64 through the inner hole 86 of the sealing tube 62, and extends inside the covering tube 64 toward the proximal end side. In the present practical embodiment, the tip end side of the covering tube 64, which is externally fitted onto the sealing tube 62, is overlapped on the proximal end face of the catheter main body 12 and is fastened by welding or other means on the outer circumferential surface of the sealing tube 62. However, as in the first practical embodiment, the tip end side of the covering tube 64 may be externally fitted and fastened to the proximal end side of the catheter main body 12.

By the sealing tube 62 and the covering tube 64 being connected to the proximal end side of the catheter main body 12, the proximal end opening of the operation lumen 28 of the catheter main body 12 is sealed, thereby preventing communication between the said operation lumen 28 and the suction lumen 26.

Moreover, regarding the operation wire 48 arranged penetrating through the operation lumen 28, the portion extending out of the proximal end opening of the operation lumen 28 passes through the sealing tube 62 and the covering tube 64, which cover the opening of the operation lumen 28, and is pulled out of the outer circumferential surface of the covering tube 64 to the outside of the catheter. Besides, the portion of the operation wire 48 that penetrates the peripheral wall portion of the catheter comprising the sealing tube 62 and the covering tube 64 is not adhered to the sealing tube 62 or the covering tube 64, thereby allowing the operation wire 48 to be pushed and pulled with respect to the catheter main body 12.

To arrange the operation wire 48 through the sealing tube 62 and the covering tube 64 that cover the operation lumen 28, a wire insertion hole 88 penetrating the sealing tube 62 and the covering tube 64 is provided. The wire insertion hole 88 can be formed by any suitable method without limitation. For example, the wire insertion hole 88 may be formed by perforating the sealing tube 62 and the covering tube 64 by after-processing, or may be mold-formed. It is acceptable as long as the sealing tube 62 and the covering tube 64 can prevent the operation lumen 28 from communicating with the suction lumen 26 and allow the suction lumen 26 to communicate with the inner hole 87 of the connection tube 14 (the covering tube 64). Therefore, for example, a slit or a notch may be formed in a corner of the sealing tube 62 for passing the operation wire 48, and the slit or the notch may be covered by the covering tube 64. It would also be acceptable that the slit or the notch may be open on the outer circumferential surface of the covering tube 64. When sealing tube 62 and the covering tube 64 are thermally welded to the proximal end side of catheter main body 12, resin melting that occurs during the thermal welding can be utilized to form insertion hole for the operation wire 48, to seal the slit or the notch formed in advance, and the like.

In this way, by adopting the structure in which the base end side of the operation wire 48, which extends out of the proximal end opening of the operation lumen 28 formed in the catheter main body 12, is taken out to the outside through the sealing tube 62 and the covering tube 64 as in the present practical embodiment, it is not necessary to form a through hole in the catheter main body 12 for taking out the operation wire 48. In particular, when a braided tube having the braided layer 16 is adopted as the catheter main body 12, the formation of a through hole in the braided tube, which is difficult to process, is obviated. This may achieve simple manufacturing process, improved product reliability, and the like.

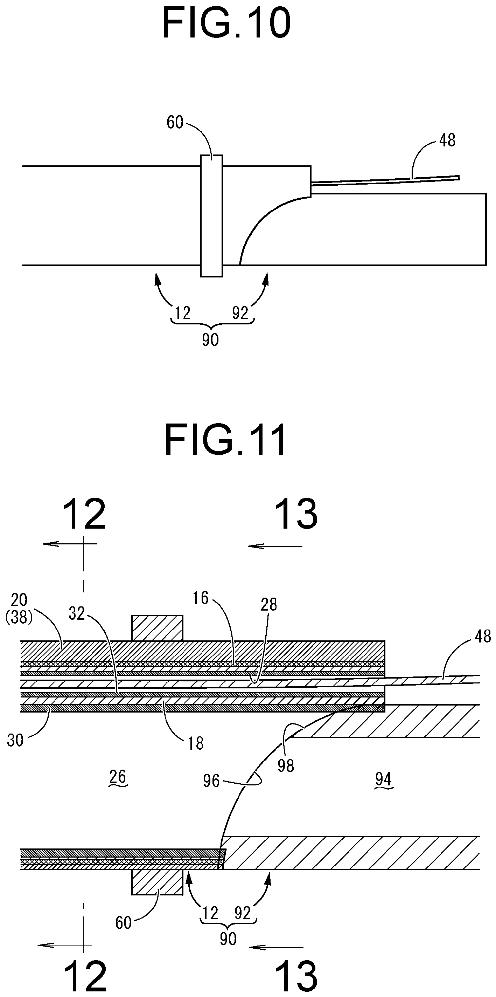

FIGS. 10 to 13 show a catheter 90 according to a fourth practical embodiment of the present invention. The catheter 90 is a suction catheter having the function similar to that of the first practical embodiment. Regarding the operation wire 48 arranged penetrating through the operation lumen 28, the catheter 90 illustrates an embodiment different from that of the first practical embodiment with respect to the structure for taking the wire out of the catheter.

That is, in the present practical embodiment, a connection tube 92 is fastened and connected to the proximal end part of the catheter main body 12, and the suction lumen 26 of the catheter main body 12 communicates with an inner hole 94 of the connection tube 92 on the proximal end side thereof.

The connection tube 92 of the present practical embodiment is overlapped on the proximal end side of the catheter main body 12 in a butted state and is fastened thereto by adhering, welding, or the like. Here, unlike the connection tube of the first practical embodiment (the sealing tube 62 and the covering tube 64), the end face of the connection tube 92 is partially butted against and overlapped on the proximal end face of the catheter main body 12. In FIG. 11, the catheter main body 12 and the connection tube 92 are shown partially overlapping each other in the fastened portion in the butted state. Here, the said overlapping portions are integrated to be fastened to each other by thermal welding of the catheter main body 12 and the connection tube 92.

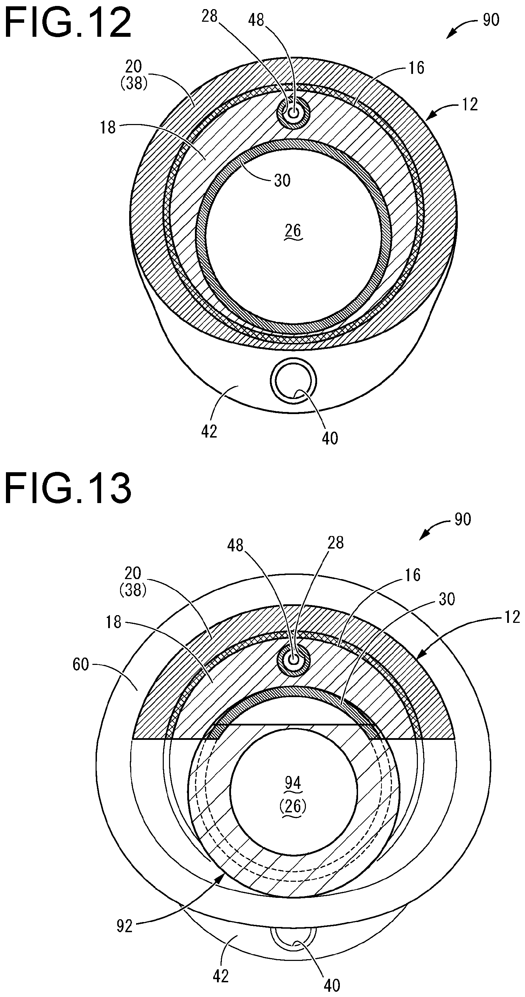

Specifically, as can be seen in FIGS. 11 and 13, the connection tube 92 extends in the length direction with an approximately constant transverse cross section, but its outer diameter dimension (the size of the transverse cross section) is smaller than that of the catheter main body 12. The distal end face of the connection tube 92 is overlapped in the butted state on a part of the proximal end face of the catheter main body 12, not an entirety thereof.

Specifically, the distal end face of the connection tube 92 is butted against and overlapped on the proximal end face of the catheter main body 12 at a location off the opening of the operation lumen 28 and on the opening peripheral edge of the suction lumen 26. With this arrangement, in the state where the connection tube 92 is connected and fastened to the catheter main body 12 in the length direction, the suction lumen 26 of the catheter main body 12 communicates with the inner hole 94 of the connection tube 92, while the operation lumen 28 of the catheter main body 12 is open to the outside on the outer circumferential surface of the connection tube 92 on the base end side of the catheter main body 12.

With this configuration, in the connected portion between the catheter main body 12 and the connection tube 92, it is possible to prevent communication between the operation lumen 28 and the suction lumen 26 while allowing the suction lumen 26 to communicate with the inner hole 94 of the connection tube 92. Besides, the operation wire 48 inserted through the operation lumen 28 of the catheter main body 12 can be easily taken out of the catheter without the need of perforating the peripheral wall of the operation lumen 28 (see the first practical embodiment, etc.).

In the present practical embodiment in particular, as shown in FIG. 11, at the proximal end part of the catheter main body 12, the opening portion (the opening peripheral edge) of the suction lumen has an obliquely cut shape so as to form an inclined end surface 96. The inclined end surface 96 has a curved shape in the side view, but may extend in an obliquely straight line, and the specific inclined shape is not limited. Besides, the distal end face of the connection tube 92 comprises an inclined end surface 98 with an inclined shape corresponding to the inclined end surface 96 of the catheter main body 12 on the proximal end side. Accordingly, the distal end face of the connection tube 92 is overlapped and fastened to the proximal end face of the catheter main body 12 over the entire circumference of the inner hole 94.

Indeed, since the inclined end surface 96 of the present practical embodiment has a curved shape that is approximately arcuate, at the opening end face (the opening peripheral edge) of the suction lumen 26, the inclination angle of the inclined end surface 96 (the inclination angle with respect to the axis-perpendicular line) is made large on the operation lumen 28 side (the upper side in FIG. 11). With this configuration, the inner circumferential surface of the suction lumen 26 is located on the inclined end surface 96 so as to spread in the axial direction, thereby surely obtaining the fastened area by being butted against the connection tube 92, while performing diametrical alignment with the connection tube 92 easily, accurately, and more reliably.

On the other hand, on the side opposite to the operation lumen 28 (the lower side in FIG. 11), the inclination angle of the inclined end surface 96 is made small, so that the axial end face of the suction lumen 26 is located on the inclined end surface 96 so as to spread in the approximately axis-perpendicular direction. With this configuration, the fastened faces, which are overlapped by the catheter main body 12 and the connection tube 92 being butted against each other, come into mutual abutment in the approximately axial direction. This makes it possible to perform axial alignment by the connection tube 92 being butted against the axial end face of the suction lumen 26 easily, accurately, and more reliably.

While the present invention has been described in detail hereinabove in terms of the practical embodiments, the invention is not limited by the specific description thereof. For example, in the preceding first through eighth preferred embodiments described in the "MEANS FOR SOLVING THE PROBLEM" section above, the catheter is not limited to a suction catheter, and the main lumen does not have to be a suction lumen. Specifically, for example, a main lumen for insertion of forceps or other treatment tools, an endoscope, or the like can be provided.

In the preceding third through sixth preferred embodiments, the operation lumen is not essential. For example, it would also be acceptable to adopt a multi-lumen structure including a sub-lumen through which forceps, an endoscope, or the like is inserted instead of the operation wire, or to adopt a single-lumen structure including the main lumen only.

In the preceding first, second, seventh through eleventh, fifteenth, and sixteenth preferred embodiments, the catheter main body does not necessarily have to be composed of the braided tube, and does not have to include the braided layer. The braided layer preferably has a circular cross section, but may have a cross section other than a circular one, for example, a cross section of oval shape or some other shape.

In the preceding third through sixteenth preferred embodiments, the fixing structure of the operation wire in the catheter main body is not limited to the adhered structure with respect to the distal end tube. For example, it would also be acceptable to fix the tip part of the operation wire to a pull ring provided at the tip end part of the catheter main body.

In the preceding first, second, and seventh through sixteenth preferred embodiments, the heat shrinkable tube and the covering layer are not essential and may be omitted. In particular, if the braided layer is omitted in the first, and sixth through ninth preferred embodiments, the heat shrinkable tube and the covering layer, which function to prevent exposure of the tip end of the braided layer, can also be omitted.

The covering layer is preferably composed of the tubular part of the distal end tip as in the preceding practical embodiments. However, the covering layer can also be composed, for example, of an independent tube that is separate from the distal end tip.

In the preceding first through sixth, and ninth through sixteenth preferred embodiments, the controller is not essential. In particular, in the third through sixth preferred embodiments, if no operation wire is provided, the controller is dispensable. Besides, the construction of the positioning mechanism for positioning the catheter and the housing is not limited to the one realized by the abutment between the stopper protrusion and the stopper abutting part, as shown in the preceding practical embodiment. Specifically, for example, the catheter may be positioned by being clasped and restrained by the housing, or the catheter may be fastened to the housing by means such as adhesion to provide the positioning mechanism.

In the preceding first through eighth, thirteenth, and fourteenth preferred embodiments, the connection tube including the sealing tube and the covering tube is not essential. Besides, in the preceding fifteenth and sixteenth preferred embodiments, a generally single connection tube can be adopted to be connected to the catheter main body without needing the sealing tube. In particular, in the preceding first through eighth preferred embodiments, if the main lumen is not a suction lumen, the sealing structure realized by the sealing tube is dispensable. Indeed, in the preceding first through eighth, thirteenth, and fourteenth preferred embodiments, the connection tube can be omitted, in which case, another component such as a hub is connected to the base end of the catheter main body without interposing the connection tube. Moreover, for example, the base end opening of the operation lumen can be covered by a covering tube serving as the connection tube without using the sealing tube. Furthermore, for example, it is not essential to adopt the structure in which the tip end side of the covering tube extends out to cover the outer circumferential surfaces of the sealing tube and the proximal end of the catheter main body.

In the preceding first through twelfth, fifteenth, and sixteenth preferred embodiments, even when the braided layer is adopted, the arrangement of the lumen with respect to the said braided layer is not particularly limited. For example, the sub-lumen such as the operation lumen may be provided in the external region of the braided layer.

Regarding the structures shown in the preceding first, third, seventh, ninth, thirteenth, and fifteenth preferred embodiments, only one of them may be adopted, or at least two of them may be adopted in combination.

### KEYS TO SYMBOLS

- 10: catheter (first practical embodiment)
- 12: catheter main body
- 14: connection tube
- 16: braided layer
- 18: inside resin layer
- 20: outside resin layer
- 22: braided tube
- 24: internal region
- 26: suction lumen (main lumen)
- 28: operation lumen
- 30: coating layer
- 32: coating layer
- 34: external region
- 36: distal resin layer
- 38: proximal resin layer
- 40: guide wire lumen
- 42: projecting part
- 44: coating layer
- 46: distal end tube
- 48: operation wire
- 50: port
- 52: adhesive
- 54: heat shrinkable tube
- 56: distal end tip
- 58: tubular part
- 60: stopper protrusion
- 62: sealing tube
- 64: covering tube
- 66: controller
- 68: catheter assembly
- 70: housing
- 72: operating part
- 74: anti-slip projection
- 76: unlocking button
- 78: stopper abutting part
- 80: catheter (second practical embodiment )
- 82: bent part
- 84: catheter (third practical embodiment )
- 86: inner hole
- 87: inner hole
- 88: wire insertion hole
- 90: catheter (fourth practical embodiment )
- 92: connection tube
- 94: inner hole
- 96: inclined end surface (catheter main body)
- 98: inclined end surface (connection tube)

## Claims

1. A catheter comprising:
a main lumen;
an operation lumen;
an operation wire inserted through the operation lumen, the operation wire being fixed on a distal end side such that the catheter is operable; and
a distal end tube fastened in a butted state to a periphery of a distal end opening of the main lumen, wherein
the operation wire extending out of a distal end opening of the operation lumen is adhered to an outer circumferential surface of the distal end tube.

2. The catheter according to claim 1, wherein a tip end part of the operation wire adhered to the outer circumferential surface of the distal end tube has a bent shape.

3. A catheter comprising
a braided tube forming a lumen, the braided tube comprising:
a braided layer;
an inside resin layer covering a radially inner side of the braided layer; and
an outside resin layer covering a radially outer side of the braided layer, wherein
at a distal end of the braided layer, the braided layer is not covered by the outside resin layer while a heat shrinkable tube is directly overlapped and fitted on the braided layer, and
a covering layer made of resin covers the heat shrinkable tube.

4. The catheter according to claim 3, wherein the covering layer comprises a tubular part on a base end side of a distal end tip.

5. The catheter according to claim 3 or 4, wherein the braided tube comprises a main lumen and an operation lumen, and the braided layer surrounds both the main lumen and the operation lumen.

6. The catheter according to claim 5, further comprising:
a distal end tube fastened in a butted state to a periphery of a distal end opening of the main lumen; and
an operation wire extending out of a distal end opening of the operation lumen and adhered to an outer circumferential surface of the distal end tube, wherein
a portion of the distal end tube where the operation wire is adhered is covered by the heat shrinkable tube.

7. A catheter assembly comprising:
a catheter comprising a main lumen and an operation lumen; and
a controller attached to a proximal end side of the catheter, the controller comprising a housing and an operating part, the operating part applying operating force to a proximal end side of an operation wire inserted through the operation lumen, wherein
the housing of the controller includes a positioning mechanism positioning the catheter with respect to the housing in a length direction in a portion where the catheter is retracted into the housing.

8. The catheter assembly according to claim 7, wherein the positioning mechanism comprises:
a stopper protrusion projecting from an outer circumferential surface of the catheter; and
a stopper abutting part provided to the housing of the controller, the stopper abutting part abutting against the stopper protrusion to define a moving end of the catheter.

9. A catheter comprising:
a catheter main body comprising a suction lumen and an operation lumen, the operation lumen allowing an operation wire to be inserted; and
a connection tube for connection with another member such as a connection hub, the connection tube being fastened to a proximal end side of the catheter main body, wherein
a sealing tube is fastened to a proximal end face of the catheter main body by being overlapped in a butted state on a periphery of an opening of the suction lumen such that a proximal end opening of the operation lumen is shut off from the suction lumen by the sealing tube, while the suction lumen communicates with the connection tube through an inner hole of the sealing tube,
a covering tube is fitted externally astride the catheter main body and the sealing tube and covers outer circumferential surfaces of the catheter main body and the sealing tube, and
the connection tube comprises the sealing tube and the covering tube.

10. The catheter according to claim 9, wherein
the proximal end opening of the operation lumen is covered by the sealing tube, and
the operation wire is taken out of the operation lumen at a location on a tip end side with respect to the covering tube.

11. The catheter according to claim 9, wherein the operation wire extending out of the proximal end opening of the operation lumen passes through the sealing tube and the covering tube and is taken out of an outer circumferential surface of the covering tube.

12. The catheter according to claim 11, wherein the catheter main body further comprises a braided layer provided in an embedded state and surrounding both the suction lumen and the operation lumen, and the suction lumen and the operation lumen are provided in an internal region surrounded by the braided layer.

13. A catheter comprising a braided tube, wherein
a suction lumen subjected to negative pressure and an operation lumen allowing an operation wire to be inserted are provided and extend in parallel,
a braided layer is provided in an embedded state and surrounds both the suction lumen and the operation lumen, the suction lumen and the operation lumen being provided in an internal region surrounded by the braided layer, and
a guide wire lumen configured to allow a guide wire to be inserted is provided in an external region outside the internal region surrounded by the braided layer.

14. The catheter according to claim 13, wherein
a portion forming the suction lumen and the operation lumen comprises the braided tube having a circular outer circumferential surface, the braided tube comprising:
an inside resin layer covering a radially inner side of the braided layer; and
an outside resin layer covering a radially outer side of the braided layer, and
a region forming the guide wire lumen projects from the circular outer circumferential surface of the braided tube.

15. A catheter comprising:
a catheter main body comprising a suction lumen and an operation lumen, the operation lumen allowing an operation wire to be inserted; and
a connection tube for connection with another member such as a connection hub, the connection tube being fastened to a proximal end side of the catheter main body, wherein
a distal end part of the connection tube is fastened to a proximal end part of the catheter main body by being overlapped in a butted state at a location off an opening of the operation lumen,
the suction lumen of the catheter main body communicates with the connection tube, and
the operation lumen of the catheter main body is open on a base end side of the catheter main body.

16. The catheter according to claim 15, wherein
at the proximal end part of the catheter main body, a proximal end opening portion of the suction lumen has an obliquely cut shape such that the proximal end opening portion of the suction lumen is shorter than a proximal end opening portion of the operation lumen, and
a distal end opening portion of the connection tube is fastened by being overlapped in the butted state via an obliquely cut shape corresponding to that of the proximal end opening portion of the suction lumen.
